# EUROPEAN PATENT SPECIFICATION

(11) **EP 0 344 425 B1**
(45) Date of publication and mention of the grant of the patent: **08.12.1993**
(21) Application number: 89105546.9
(22) Date of filing: 29.03.1989
(51) Int. Cl.: C07C 275/18, C07C 275/28, C07C 335/06, C07C 335/16, C07D 333/20, C07D 213/40, A61K 31/17

(54) **N-[[(2,6-disubstituted)phenyl]-N'- arylalkyl] ureas as antihypercholesterolemic and antiatherosclerotic agents**
N-[[(2,6-disubstituierte)phenyl]N'- arylalkyl] Harnstoffe als antihypercholesterolemische und antiatherosclerotische Mittel
N-[[(phényl 2,6 disubstitué)]N'- arylalkyl] urées comme agents antihypercholesterolemiques et antiatherosclérotiques

(30) Priority: 30.03.1988 US 175089
(43) Date of publication of application: 06.12.1989
(73) Proprietor: WARNER-LAMBERT COMPANY, Morris Plains New Jersey 07950 (US)
(72) Inventor: Trivedi, Bharat Kalidas, Canton Michigan 48187 (US)
(74) Representative: Mansmann, Ivo

(56) References cited:
- AT-B- 288 605
- DE-A- 1 642 237
- DE-B- 1 238 455
- GB-A- 2 113 684
- US-A- 3 660 484
- US-A- 4 387 106
- US-A- 4 623 662

## Description

This invention relates to chemical compounds having pharmacological activity, to pharmaceutical compositions which include these compounds, and to a pharmaceutical method of treatment. More particularly, this invention concerns certain substituted urea and thiourea compounds which inhibit the enzyme acyl-coenzyme A:cholesterol acyltransferase (ACAT), pharmaceutical compositions containing these compounds, and a method of use of these compounds for the manufacturing of pharmaceuticals for treating hypercholesterolemia and atherosclerosis.

In recent years the role which elevated blood plasma levels of cholesterol plays in pathological conditions in man has received much attention. Deposits of cholesterol in the vascular system have been indicated as causative of a variety of pathological conditions including coronary heart disease.

Initially, studies of this problem were directed toward finding therapeutic agents which would be effective in lowering total serum cholesterol levels. It is now known that cholesterol is transported in the blood in the form of complex particles consisting of a core of cholesteryl esters plus triglycerides and an exterior consisting primarily of phospholipids and a variety of types of protein which are recognized by specific receptors. For example, cholesterol is carried to the sites of deposit in blood vessels in the form of low density lipoprotein cholesterol (LDL cholesterol) and away from such sites of deposit by high density lipoprotein cholesterol (HDL cholesterol).

Following these discoveries, the search for therapeutic agents which control serum cholesterol turned to finding compounds which are more selective in their action; that is, agents which are effective in elevating the blood serum levels of HDL cholesterol and/or lowering the levels of LDL cholesterol. While such agents are effective in moderating the levels of serum cholesterol, they have little or no effect on controlling the initial absorption of dietary cholesterol in the body through the intestinal wall.

In intestinal mucosal cells, dietary cholesterol is absorbed as free cholesterol which must be esterified by the action of the enzyme acyl-CoA:cholesterol acyltransferase (ACAT) before it can be packaged into the chylomicrons which are then released into the blood stream. Thus, therapeutic agents which effectively inhibit the action of ACAT prevent the intestinal absorption of dietary cholesterol into the blood stream or the reabsorption of cholesterol which has been previously released into the intestine through the body's own regulatory action.

United States Patent 4,387,105 to DeVries, et al. discloses a method of treating atherosclerosis employing certain dialkylurea and dialkylthiourea compounds.

United States Patent 4,387,106 to DeVries, et al. discloses a method of treating atherosclerosis using certain N-phenyl- or N-[substituted(phenyl)]-N′,N′-dialkylurea and thiourea compounds.

United States Patent 4,387,106 to DeVries, et al. discloses methods for treating atherosclerosis using certain trisubstituted N-[substituted(phenyl)]-N′,N′-diarylalkyl urea and thiourea compounds.

United States Patent 4,397,868 to DeVries, et al. discloses methods for treating atherosclerosis using certain trisubstituted urea compounds.

United States Patent 4,473,579 to DeVries, et al. discloses certain tetrasubstituted urea compounds and their use as agents for treating atherosclerosis.

United States Patent 4,623,662 to DeVries discloses a method of reducing arterial wall deposits of cholesterol employing certain trisubstituted urea and thiourea compounds.

The present invention provides a class of compounds with ACAT inhibitory activity having the structure 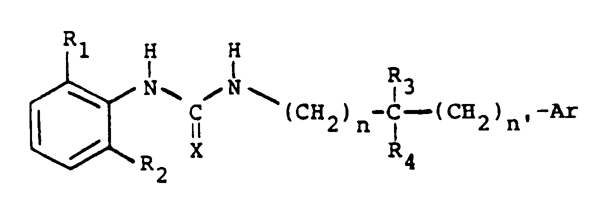
wherein R₁ and R₂ are independently selected from alkyl or alkoxy of from one to six carbons, n is zero or an integer of from one to four, when n is zero, n′ is an integer of from one to four, otherwise n′ is zero, and X is oxygen or sulfur. R₃ and R₄ are independently selected from hydrogen or alkyl of from one to six carbon atoms.

Ar is phenyl, 1- or 2-naphthyl, 2- or 3-thienyl, or 2-, 3-, or 4-pyridinyl any of which may be unsubstituted or substituted by alkyl of from one to six carbon atoms, hydroxy, alkoxy of from one to six carbon atoms, benzyloxy, fluorine, chlorine, bromine, nitro, trifluoromethyl, -NR₅R₆ in which R₅ and R₆ are independently hydrogen or alkyl of from one to six carbon atoms, or -NH-acetyl.

The compounds of the present invention form a class of substituted ureas and thioureas having potent activity as inhibitors of the enzyme acyl CoA: cholesterol acyltransferase (ACAT) and thus are useful as agents for the treatment of hypercholesterolemia and atherosclerosis.

In the compounds of the present invention, the first nitrogen atom of the urea or thiourea moiety is monosubstituted by a phenyl group which is substituted in the 2- and 6-positions by alkyl or alkoxy groups. Preferred compounds of this invention are the 2,6-dialkylsubstituted urea compounds, with 2,6-bis(1-methylethyl) being the most preferred.

The second nitrogen atom of the urea or thiourea moiety of compounds of this invention is monosubstituted with an arylalkyl group in which the aryl groups may be unsubstituted phenyl, naphthyl, thienyl, or pyridinyl. Alternatively the aryl groups may be substituted with alkyl of from one to six carbon atoms, hydroxy, alkoxy of from one to six carbon atoms, benzyloxy, fluorine, chlorine, bromine, nitro, trifluoromethyl, -NR₅R₆ in which R₆ and R₆ are independently hydrogen or alkyl of from one to six carbon atoms, or -NH-acetyl. Preferred compounds of this invention are those in which Ar is unsubstituted phenyl or naphthyl, and R₃ and R₄ are alkyl of from one to six carbon atoms.

Examples of compounds contemplated as falling within the scope of the invention are the following:
N-[2,6-bis(1-methylethyl)phenyl]-N′-(2-phenyl-2-propylpentyl)urea;
N-[2,6-bis(1-methylethyl)phenyl]-N′-(2-ethyl-2-phenylbutyl)urea;
N-[2,6-bis(1-methylethyl)phenyl]-N′-(2-methyl-2-phenylpropyl)urea;
N-[2,6-bis(1-methylethyl)phenyl]-N′-(3,3-dimethyl-2-phenylbutyl)urea;
N-[2,6-bis(1-methylethyl)phenyl]-N′-(2-butyl-2-phenylhexyl)urea;
N-[2,6-bis(1-methylethyl)phenyl]-N′-(2-phenylethyl)urea;
N-[2,6-bis(1-methylethyl)phenyl]-N′-(2-phenylpropyl)urea;
N-[2,6-bis(1-methylethyl)phenyl]-N′-(3-phenylpropyl)urea;
N-[2,6-bis(1-methylethyl)phenyl]-N'-[2-(1-naphthalenyl)ethyl]urea;
[R]-N-[2,6-bis(1-methylethyl)phenyl]-N'-[1-(1-naphthalenyl)ethyl]urea; and
[S]-N-[2,6-bis(1-methylethyl)phenyl]-N'-[1-(1-naphthalenyl)ethyl]urea.

By the term "alkyl" as used throughout this specification and the appended claims is meant a branched or unbranched hydrocarbon grouping derived from a saturated hydrocarbon of from one to six carbon atoms by removal of a single hydrogen atom. Examples of alkyl groups contemplated as falling within the scope of this invention include methyl, ethyl, propyl, 1-methylethyl, butyl, 1-methylpropyl, 2-methylpropyl, and 1,1-dimethylethyl.

By the term "alkoxy" is meant an alkyl group, as defined above, attached to the parent molecular moiety through an oxygen atom.

In those instances where the compounds of the present invention bear a basic nitrogen atom, the compounds are capable of forming acid addition salts. These acid addition salts are also contemplated as falling within the scope of this invention.

While the acid addition salts may vary from the free base form of the compounds in certain properties such as melting point and solubility, they are considered equivalent to the free base forms for the purposes of this invention.

The acid addition salts may be generated from the free base forms of the compounds by reaction of the latter with one equivalent of a suitable nontoxic, pharmaceutically acceptable acid, followed by evaporation of the solvent employed for the reaction and recrystallisation of the salt, if required. The free base may be recovered from the acid addition salt by reaction of the salt with a water solution of the salt with a suitable base such as sodium carbonate, sodium bicarbonate, potassium carbonate, sodium hydroxide, and the like.

Suitable acids for forming acid addition salts of the compounds of this invention include, but are not necessarily limited to acetic, benzoic, benzenesulfonic, tartaric, hydrobromic, hydrochloric, citric, fumaric, gluconic, glucuronic, glutamic, lactic, malic, maleic, methanesulfonic, pamoic, salicylic, stearic, succinic, sulfuric, and tartaric acids. The class of acids suitable for the formation of nontoxic, pharmaceutically acceptable salts is well known to practitioners of the pharmaceutical formulation arts. (See, for example, Stephen N. Berge, et al. J. Pharm. Sciences, **66**:1-19 (1977).

The compounds of the present invention may also exist in different stereoisomeric forms by virtue of the presence of one or more asymmetric centers in the compound. The present invention contemplates all stereoisomeric forms of the compounds as well as mixtures thereof, including racemic mixtures. Individual stereoisomers may be obtained, if desired by methods known in the art as, for example, the separation of stereoisomers on chiral chromatographic columns.

Further, the compounds of this invention may exist in unsolvated as well as solvated forms with pharmaceutically acceptable solvents such as water, ethanol and the like. In general, the solvated forms are considered equivalent to the unsolvated forms for the purposes of this invention.

The compounds of the present invention are prepared by reacting the appropriately 2,6-disubstituted isocyanate or thioisocyanate with the desired amine. The starting materials are generally known or commercially available materials or, if not previously known, are synthesized by methods generally known in the art.

The reaction is generally carried out in a polar aprotic organic solvent such as ethyl acetate, at any temperature between room temperature and the boiling point of the solvent, with room temperature being preferred.

The reaction is allowed to proceed until analysis of the mixture by a means such as chromatography indicates that the reaction is substantially complete. Reaction times may vary between about two hours to about 24 hours, depending upon the particular reagents and reaction temperature employed. The starting isocyanate or thioisocyanate compounds are known or commercially available or, if not previously known, are prepared by methods well known in the art from the corresponding amine compounds.

As shown by the data presented below in Table 1, the compounds of the present invention are potent inhibitors of the enzyme acyl-CoA:cholesterol acyltransferase (ACAT), and are thus effective in inhibiting the esterification and transport of cholesterol across the intestinal cell wall. The compounds of the present invention are thus useful in pharmaceutical formulations for the treatment of hypercholesterolemia or atherosclerosis.

### In Vitro Tests

The ability of representative compounds of the present invention to inhibit ACAT was measured using an in vitro test more fully described in Field, F. J. and Salone, R. G., Biochemica et Biophysica **712**:557-570 (1982). The test assesses the ability of a test compound to inhibit the acylation of cholesterol by oleic acid by measuring the amount of radio-labeled cholesterol oleate formed from radio-labeled oleic acid in a tissue preparation containing rabbit intestinal microsomes.

The data appear in Table 1 where they are expressed as IC₅₀ values; i.e. the concentration of test compound required to inhibit 50% expression of the enzyme.

In therapeutic use as agents for treating hypercholesterolemia or atherosclerosis, the compounds utilized in the pharmaceutical method of this invention are administered to the patient at dosage levels of from 250 to 1000 mg per day. For a normal human adult of approximately 70 kg of body weight, this translates into a dosage of from 5 to 20 mg/kg of body weight per day. The specific dosages employed, however, may be varied depending upon the requirements of the patient, the severity of the condition being treated, and the activity of the compound being employed. The determination of optimum dosages for a particular situation is within the skill of the art.

**Table 1**

| Compound of Example | IC₅₀ (µM) |
|---|---|
| 1 | 0.030 |
| 2 | 0.017 |
| 3 | 0.026 |
| 4 | 0.016 |
| 5 | 0.030 |
| 6 | 0.088 |
| 7 | 0.035 |
| 8 | 0.191 |
| 9 | 0.041 |
| 10 | 0.039 |
| 11 | 0.035 |
| 12 | 0.630 |

For preparing pharmaceutical compositions from the compounds of this invention, inert, pharmaceutically acceptable carriers can be either solid or liquid. Solid form preparations include powders, tablets, dispersible granules, capsules, and cachets.

A solid carrier can be one or more substances which may also act as diluents, flavoring agents, solubilizers, lubricants, suspending agents, binders, or tablet disintegrating agents; it can also be an encapsulating material.

In powders, the carrier is a finely divided solid which is in a mixture with the finely divided active component. In tablets, the active compound is mixed with the carrier having the necessary binding properties in suitable proportions and compacted in the shape and size desired.

Powders and tablets preferably contain between about 5 to about 70% by weight of the active ingredient. Suitable carriers are magnesium carbonate, magnesium stearate, talc, lactose, sugar, pectin, dextrin, starch, tragacanth, methyl cellulose, sodium carboxymethyl cellulose, a low-melting wax, cocoa butter, and the like.

The term "preparation" is intended to include the formulation of the active compound with encapsulating material as a carrier providing a capsule in which the active component (with or without other carriers) is surrounded by a carrier, which is thus in association with it. In a similar manner, cachets are also included.

Tablets, powders, cachets, and capsules can be used as solid dosage forms suitable for oral administration.

Liquid form preparations include solutions suitable for oral administration, or suspensions and emulsions suitable for oral administration. Aqueous solutions for oral administration can be prepared by dissolving the active compound in water and adding suitable flavorants, coloring agents, stabilizers, and thickening agents as desired. Aqueous suspensions for oral use can be made by dispersing the finely divided active component in water together with a viscous material such as natural or synthetic gums, resins, methyl cellulose, sodium carboxymethyl cellulose, and other suspending agents known to the pharmaceutical formulation art.

Preferably, the pharmaceutical preparation is in unit dosage form. In such form, the preparation is divided into unit doses containing appropriate quantities of the active component. The unit dosage form can be a packaged preparation, the package containing discrete quantities of the preparation, for example, packeted tablets, capsules, and powders in vials or ampoules. The unit dosage form can also be a capsule, cachet, or tablet itself, or it can be the appropriate number of any of these packaged forms.

The following preparative examples are provided to enable one skilled in the art to practice the invention, and are illustrative thereof. They are not to be read as limiting the scope of the invention as it is defined by the appended claims.

### Example 1

### Preparation of N-[2,6-bis(1-methylethyl)phenyl]-N′-(2-phenyl-2-propylpentyl)urea

To a solution of 2,2-di-n-propylphenethylamine (1.10 g, 0.005 mole) in 50 ml of ethyl acetate was added 2,6-bis(1-Methylethyl)phenyl isocyanate (0.99 g, 0.005 mole). The resulting mixture was stirred at room temperature for 20 hours. The solvent was removed under vacuum, and the residue was suspended in hexane and the solids collected by filtration. The residue was oven-dried to yield 1.53 g of the title compound, mp 149-151°C.

| Analysis for C₂₇H4₀N₂O: | | | |
|---|---|---|---|
| Calc.: | C = 79.36%; | H = 9.87%; | N = 6.86% |
| Found: | C = 79.56%; | H = 10.01%; | N = 6.96%. |

### Example 2

### Preparation of N-[2,6-bis(1-methylethyl)phenyl]-N′-(2-ethyl-2-phenylbutyl)urea

The title compound, mp 170-173°C, was prepared from 2,6-bis(1-methylethyl)phenyl isocyanate and 2,2-diethylphenethylamine using the method of Example 1.

| Analysis for C₂₅H₃₆N₂O: | | | |
|---|---|---|---|
| Calc.: | C = 78.90%; | H = 9.53%; | N = 7.36% |
| Found: | C = 79.28%; | H = 9.71%; | N = 7.63%. |

### Example 3

### Preparation of N-[2,6-bis(1-methylethyl)phenyl]-N′-(2-methyl-2-phenylpropyl)urea

The title compound, range 153-158°C, was prepared from 2,6-bis(1-methylethyl)phenyl isocyanate and 2,2-dimethylphenethylamine using the method of Example 1.

| Analysis for C₂₃H₃₂N₂O: | | | |
|---|---|---|---|
| Calc.: | C = 78.37%; | H = 9.15%; | N = 7.95% |
| Found: | C = 78.46%; | H = 9.32%; | N = 7.96%. |

### Example 4

### Preparation of N-[2,6-bis(1-methylethyl)phenyl]-N′-(3,3-dimethyl-2-phenylbutyl)urea

The title compound, mp 176-178°C, was prepared from 2,6-bis(1-methylethyl)phenyl isocyanate and 2-tert-butylphenethylamine using the method of Example 1.

| Analysis for C₂₅H₃₆N₂O: | | | |
|---|---|---|---|
| Calc.: | C = 78.90%; | H = 9.53%; | N = 7.36% |
| Found: | C = 78.62%; | H = 9.45%; | N = 7.21%. |

### Example 5

### Preparation of N-[2,6-bis(1-methylethyl)phenyl]-N′-(2-butyl-2-phenylhexyl)urea

The title compound, melting range 76-81°C, was prepared from 2,6-bis(1-methylethyl)phenyl isocyanate and 2,2-di-n-butylphenethylamine using the method of Example 1.

| Analysis for C₂₉H₄₄N₂O: | | | |
|---|---|---|---|
| Calc.: | C = 79.77%; | H = 10.16%; | N = 6.41% |
| Found: | C = 79.97%; | H = 10.41%; | N = 6.29%. |

### Example 6

### Preparation of N-[2,6-bis(1-methylethyl)phenyl]-N′-(2-phenylethyl)urea

The title compound, mp 231-233°C, was prepared from 2,6-bis(1-methylethyl)phenyl isocyanate and 2-phenethylamine using the method of Example 1.

| Analysis for C₂₁H₂₈N₂O | | | |
|---|---|---|---|
| Calc.: | C = 77.74%; | H = 8.69%; | N = 8.63% |
| Found: | C = 77.60%; | H = 8.85%; | N = 8.49%. |

### Example 7

### Preparation of N-[2,6-bis(1-methylethyl)phenyl]-N′-(2-phenylpropyl)urea

The title compound, mp 175-176°C, was prepared from 2,6-bis(1-methylethyl)phenyl isocyanate and 2-methyl-2-phenethylamine using the method of Example 1.

| Analysis for C₂₂H₃₀N₂O | | | |
|---|---|---|---|
| Calc.: | C = 78.06%; | H = 8.93%; | N = 8.27% |
| Found: | C = 78.12%; | H = 9.02%; | N = 8.18%. |

### Example 8

### Preparation of N-[2,6-bis(1-methylethyl)phenyl]-N'-(3-phenylpropyl)urea

The title compound, mp 205-207°C, was prepared from 2,6-bis(1-methylethyl)phenyl isocyanate and 3-phenyl-propylamine using the method of Example 1.

| Analysis for C₂₂H₃₀N₂O | | | |
|---|---|---|---|
| Calc.: | C = 78.06%; | H = 8.93%; | N = 8.27% |
| Found: | C = 77.95%; | H = 9.09%; | N = 8.27%. |

### Example 9

### Preparation of N-[2,6-bis(1-methylethyl)phenyl]-N'-[2-(1-naphthalenyl)ethyl]urea

The title compound, mp 174-176°C, was prepared from 2,6-bis(1-methylethyl)phenyl isocyanate and 2-(1-naphthyl)ethylamine using the method of Example 1.

| Analysis for C₂₅H₃₀N₂O | | | |
|---|---|---|---|
| Calc.: | C = 80.17%; | H = 8.07%; | N = 7.47% |
| Found: | C = 80.25%; | H = 8.42%; | N = 7.42%. |

### Example 10

### Preparation of N-[2,6-bis(1-methylethyl)phenyl]-N'-[2-(2-naphthalenyl)ethyl]urea

The title compound, mp 207-209°C, was prepared from 2,6-bis(1-methylethyl)phenyl isocyanate and 2-(2-naphthyl)ethylamine using the method of Example 1.

| Analysis for C₂₅H₃₀N₂O | | | |
|---|---|---|---|
| Calc.: | C = 80.17%; | H = 8.07%; | N = 7.47% |
| Found: | C = 79.99%; | H = 8.09%; | N = 7.31%. |

### Example 11

### Preparation of [R]-N-[2,6-bis(1-methylethyl) phenyl]-N'-[1-(1-naphthalenyl)ethyl]urea

The title compound, mp 215-217°C was prepared from 2,6-bis(1-methylethyl)phenyl isocyanate and optically pure [R]-1-(1-naphthyl)ethylamine using the method of Example 1.
[α]_{D}²⁴ = -23.7° (c = 1.13% in methanol).

| Analysis for C₂₅H₃₀N₂O: | | | |
|---|---|---|---|
| Calc.: | C = 80.17%; | H = 8.07%; | N = 7.47% |
| Found: | C = 80.14%; | H = 8.05%; | N = 7.29%. |

### Example 12

### Preparation of [S]-N-[2,6-bis(1-methylethyl) phenyl]-N'-[1-(1-naphthalenyl)ethyl]urea

The title compound, mp 215-217°C was prepared from 2,6-bis(1-methylethyl)phenyl isocyanate and optically pure [S]-1-(1-naphthyl)ethylamine using the method of Example 1.
[α]_{D}²⁴ = +22.9° (c = 1.17% in methanol).

| Analysis for C₂₅H₃₀N₂O: | | | |
|---|---|---|---|
| Calc.: | C = 80.17%; | H = 8.07%; | N = 7.47% |
| Found: | C = 79.94%; | H = 8.00 %; | N =7.27 %. |

## Claims

1. A compound having the structural formula 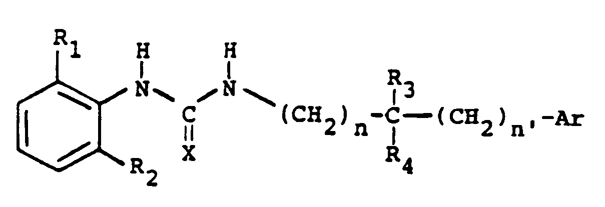 wherein R₁ and R₂ are independently selected from
alkyl of from one to six carbon atoms, or
alkoxy of from one to six carbon atoms;
n is zero or an integer of from one to four;
n' is an integer of from one to four when n is zero, otherwise n' is zero;
X is oxygen or sulfur;
R₃ and R₄ are independently selected from hydrogen, or
alkyl of from one to six carbon atoms;
Ar is
unsubstituted phenyl, 1- or 2-naphthyl, 2- or 3-thienyl, 2-, 3-, or 4-pyridinyl, or
phenyl, 1- or 2-naphthyl, 2- or 3-thienyl, 2-, 3-, or 4-pyridinyl, substituted with
alkyl of from one to six carbon atoms,
hydroxy,
alkoxy of from one to six carbon atoms,
benzyloxy,
fluorine,
chlorine,
bromine,
nitro,
trifluoromethyl,
-NR₅R₆ in which R₅ and R₆ are independently
hydrogen or alkyl of from one to six carbon atoms, or
-NH-acetyl;
or a pharmaceutically acceptable salt thereof.

2. A compound as defined by Claim 1 wherein R₁ and R₂ are alkyl of from one to six carbon atoms.

3. A compound as defined by Claim 2 wherein R₁ and R₂ are 1-methylethyl.

4. A compound as defined by Claim 1 selected from the group consisting of
N-[2,6-bis(1-methylethyl)phenyl]-N'-(2-phenyl-2-propylpentyl)urea;
N-[2,6-bis(1-methylethyl)phenyl]-N'-(2-ethyl-2-phenylbutyl)urea;
N-[2,6-bis(1-methylethyl)phenyl]-N'-(2-methyl-2-phenylpropyl)urea;
N-[2,6-bis(1-methylethyl)phenyl]-N'-(3,3-dimethyl-2-phenylbutyl)urea;
N-[2,6-bis(1-methylethyl)phenyl]-N'-(2-butyl-2-phenylhexyl)urea;
N-[2,6-bis(1-methylethyl)phenyl]-N'-(2-phenylethyl)urea;
N-[2,6-bis(1-methylethyl)phenyl]-N'-(2-phenylpropyl)urea;
N-[2,6-bis(1-methylethyl)phenyl]-N'-(3-phenylpropyl)urea;
N-[2,6-bis(1-methylethyl)phenyl]-N'-[2-(1-naphthalenyl)ethyl]urea;
[R]-N-[2,6-bis(1-methylethyl)phenyl]-N'-[1-(1-naphthalenyl)ethyl]urea; and
[S]-N-[2,6-bis(1-methylethyl)phenyl]-N'-[1-(1-naphthalenyl)ethyl]urea.

5. A pharmaceutical composition comprising an ACAT-inhibitory effective amount of a compound according to Claims 1 to 4 in combination with a pharmaceutically acceptable carrier.

6. Use of a compound according to Claims 1 to 4 for the manufacturing of pharmaceuticals for treating hypercholesterolemia or atherosclerosis.

7. A process for preparing a compound having the structural formula 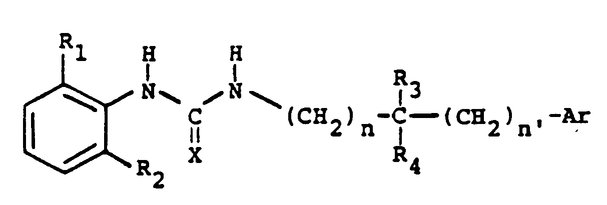 wherein R₁ and R₂ are independently selected from
alkyl of from one to six carbon atoms, or
alkoxy of from one to six carbon atoms;
n is zero or an integer of from one to four;
n' is an integer of from one to four when n is zero, otherwise, n' is zero;
X is oxygen or sulfur;
R₃ and R₄ are independently selected from hydrogen, or
alkyl of from one to six carbon atoms;
Ar is
unsubstituted phenyl, 1- or 2-naphthyl, 2- or 3-thienyl, 2-, 3-, or 4-pyridinyl, or
phenyl, 1- or 2-naphthyl, 2- or 3-thienyl, 2-, 3-, or 4-pyridinyl, substituted with
alkyl of from one to six carbon atoms,
hydroxy,
alkoxy of from one to six carbon atoms,
benzyloxy,
fluorine,
chlorine,
bromine,
nitro,
trifluoromethyl,
-NR₅R₆ in which R₅ and R₆ are independently
hydrogen or alkyl of from one to six carbon atoms, or
-NH-acetyl;
or a pharmaceutically acceptable salt thereof;
comprising the steps of reacting an isocyanate or thioisocyanate of the structure 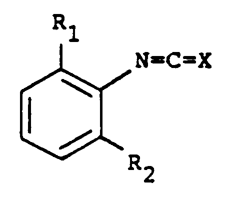 wherein R₁, R₂, and X are as defined above, with an amine having the structure 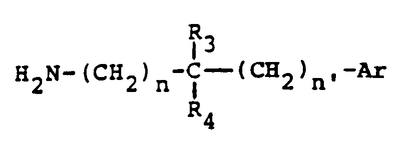 wherein n, n', R₃, R₄, and Ar are as defined above, thereafter separating the product of said reaction and converting, if desired, said product to a pharmaceutically acceptable salt by conventional methods.

8. A process for preparing a compound as defined by Claim 7 wherein R₁ and R₂ are alkyl of from one to six carbon atoms.

9. A process for preparing a compound as defined by Claim 8 wherein R₁ and R₂ are 1-methylethyl.

10. A process for preparing a compound as defined by Claim 7 selected from the group consisting of
N-[2,6-bis(1-methylethyl)phenyl]-N'-(2-phenyl-2-propylpentyl)urea;
N-[2,6-bis(1-methylethyl)phenyl]-N'-(2-ethyl-2-phenylbutyl)urea;
N-[2,6-bis(1-methylethyl)phenyl]-N'-(2-methyl-2-phenylpropyl)urea;
N-[2,6-bis(1-methylethyl)phenyl]-N'-(3,3-dimethyl-2-phenylbutyl)urea;
N-[2,6-bis(1-methylethyl)phenyl]-N'-(2-butyl-2-phenylhexyl)urea;
N-[2,6-bis(1-methylethyl)phenyl]-N'-(2-phenylethyl)urea;
N-[2,6-bis(1-methylethyl)phenyl]-N'-(2-phenylpropyl)urea;
N-[2,6-bis(1-methylethyl)phenyl]-N'-(3-phenylpropyl)urea;
N-[2,6-bis(1-methylethyl)phenyl]-N'-[2-(1-naphthalenyl)ethyl]urea;
[R]-N-[2,6-bis(1-methylethyl)phenyl]-N'-[1-(1-naphthalenyl)ethyl]urea; and
[S]-N-[2,6-bis(1-methylethyl)phenyl]-N'-[1-(1-naphthalenyl)ethyl]urea.

11. A process for manufacturing a pharmaceutical composition characterized by adding a compound manufactured according to claims 7 to 10 to a pharmaceutically acceptable solid or liquid carrier.

## Patentansprüche

1. Verbindung der Strukturformel: 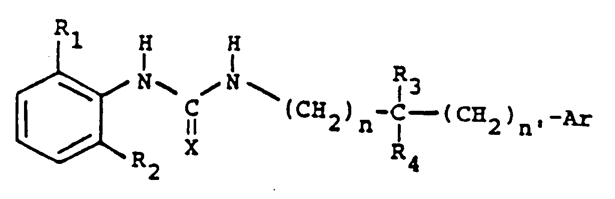 worin bedeuten:
R₁ und R₂ unabhängig jeweils Alkyl mit 1 bis 6 Kohlenstoffatomen oder Alkoxy mit 1 bis 6 Kohlenstoffatomen;
n=0 oder eine ganze Zahl von 1 bis 4;
n' eine ganze Zahl von 1 bis 4, wenn n = 0, ansonsten ist n'=0;
X Sauerstoff oder Schwefel;
R₃ und R₄ unabhängig voneinander jeweils Wasserstoff oder Alkyl mit 1 bis 6 Kohlenstoffatomen;
Ar unsubstituiertes Phenyl, 1- oder 2-Naphthyl, 2- oder 3-Thienyl, 2-, 3- oder 4-Pyridinyl oder Phenyl, 1- oder 2-Naphthyl, 2- oder 3-Thienyl, 2-, 3- oder 4-Pyridinyl, substituiert durch Alkyl mit 1 bis 6 Kohlenstoffatomen, Hydroxy, Alkoxy mit 1 bis 6 Kohlenstoffatomen, Benzyloxy, Fluor, Chlor, Brom, Nitro, Trifluormethyl, -NR₅R₆ mit R₅ und R₆ unabhängig voneinander jeweils gleich Wasserstoff oder Alkyl mit 1 bis 6 Kohlenstoffatomen, oder -NH-Acetyl;
oder ein pharmazeutisch akzeptables Salz derselben.

2. Verbindung nach Anspruch 1, worin R₁ und R₂ Alkyl mit 1 bis 6 Kohlenstoffatomen bedeuten.

3. Verbindung nach Anspruch 2, worin R₁ und R₂ für 1-Methylethyl stehen.

4. Verbindung nach Anspruch 1, ausgewählt aus:
N-[2,6-Bis-(1-methylethyl)-phenyl]-N'-(2-phenyl-2-propylpentyl)-harnstoff;
N-[2,6-Bis-(1-methylethyl)-phenyl]-N'-(2-ethyl-2-phenylbutyl)-harnstoff;
N-[2,6-Bis-(1-methylethyl)-phenyl]-N'-(2-methyl-2-phenylpropyl)-harnstoff;
N-[2,6-Bis-(1-methylethyl)-phenyl]-N'-(3,3-dimethyl-2-phenylbutyl)-harnstoff;
N-[2,6-Bis-(1-methylethyl)-phenyl]-N'-(2-butyl-2-phenylhexyl)-harnstoff;
N-[2,6-Bis-(1-methylethyl)-phenyl]-N'-(2-phenylethyl)-harnstoff;
N-[2,6-Bis-(1-methylethyl)-phenyl]-N'-(2-phenylpropyl)-harnstoff;
N-[2,6-Bis-(1-methylethyl)-phenyl]-N'-(3-phenylpropyl)-harnstoff;
N-[2,6-Bis-(1-methylethyl)-phenyl]-N'-[2-(1-naphthalenyl)-ethyl]-harnstoff;
[R]-N-[2,6-Bis-(1-methylethyl)-phenyl]-N'-[1-(1-naphthalenyl)-ethyl]-harnstoff und
[S]-N-[2,6-Bis-(1-methylethyl)-phenyl]-N'-[1-(1-naphthalenyl)-ethyl]-harnstoff.

5. Arzneimittelzubereitung mit einer ACAT-inhibierenden wirksamen Menge einer Verbindung nach Ansprüchen 1 bis 4 in Kombination mit einem pharmazeutisch akzeptablen Träger.

6. Verwendung einer Verbindung nach Ansprüchen 1 bis 4 zur Herstellung von Arzneimitteln zur Behandlung von Hypercholesterinämie oder Arteriosklerose.

7. Verfahren zur Herstellung einer Verbindung der Strukturformel: 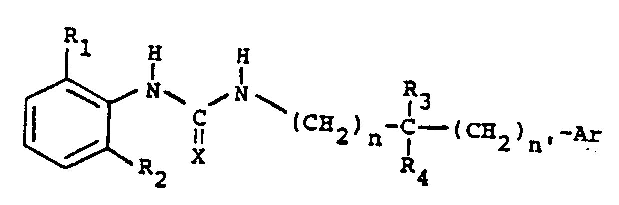 worin bedeuten:
R₁ und R₂ unabhängig jeweils Alkyl mit 1 bis 6 Kohlenstoffatomen oder Alkoxy mit 1 bis 6 Kohlenstoffatomen;
n=0 oder eine ganze Zahl von 1 bis 4;
n' eine ganze Zahl von 1 bis 4, wenn n = 0, ansonsten ist n' null;
X Sauerstoff oder Schwefel;
R₃ und R₄ unabhängig voneinander jeweils Wasserstoff oder Alkyl mit 1 bis 6 Kohlenstoffatomen;
Ar unsubstituiertes Phenyl, 1- oder 2-Naphthyl, 2- oder 3-Thienyl, 2-, 3- oder 4-Pyridinyl oder Phenyl, 1- oder 2-Naphthyl, 2- oder 3-Thienyl, 2-, 3- oder 4-Pyridinyl, substituiert durch Alkyl mit 1 bis 6 Kohlenstoffatomen, Hydroxy, Alkoxy mit 1 bis 6 Kohlenstoffatomen, Benzyloxy, Fluor, Chlor, Brom, Nitro, Trifluormethyl, -HR₅R₆ mit R₅ und R₆ unabhängig voneinander jeweils gleich Wasserstoff oder Alkyl mit 1 bis 6 Kohlenstoffatomen, oder -NH-Acetyl;
oder eines pharmazeutisch akzeptablen Salzes derselben; das die Umsetzung eines Isocyanats oder Thioisocyanats der Struktur 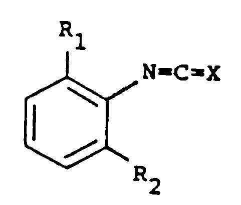 worin R₁, R₂ und X die oben angegebene Bedeutung besitzen, mit einem Amin der Struktur 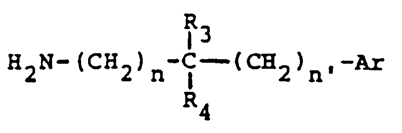 worin n, n', R₃, R₄ und Ar die oben angegebene Bedeutung besitzen, anschließende Abtrennung des Produkts dieser Reaktion und gewünschtenfalls Umwandlung des Produkts in ein pharmazeutisch akzeptables Salz in üblicher bekannter Weise, beinhaltet.

8. Verfahren zur Herstellung einer Verbindung nach Anspruch 7, worin R₁ und R₂ für Alkyl mit 1 bis 6 Kohlenstoffatomen stehen.

9. Verfahren zur Herstellung einer Verbindung nach Anspruch 8, worin R₁ und R₂ für 1-Methylethyl stehen.

10. Verfahren zur Herstellung einer Verbindung nach Anspruch 7, ausgewählt aus der Gruppe:
N-[2,6-Bis-(1-methylethyl)-phenyl]-N'-(2-phenyl-2-propylpentyl)-harnstoff;
N-[2,6-Bis-(1-methylethyl)-phenyl]-N'-(2-ethyl-2-phenylbutyl)-harnstoff;
N-[2,6-Bis-(1-methylethyl)-phenyl]-N'-(2-methyl-2-phenylpropyl)-harnstoff;
N-[2,6-Bis-(1-methylethyl)-phenyl]-N'-(3,3-dimethyl-2-phenylbutyl)-harnstoff;
N-[2,6-Bis-(1-methylethyl)-phenyl]-N'-(2-butyl-2-phenylhexyl)-harnstoff;
N-[2,6-Bis-(1-methylethyl)-phenyl]-N'-(2-phenylethyl)-harnstoff;
N-[2,6-Bis-(1-methylethyl)-phenyl]-N'-(2-phenylpropyl)-harnstoff;
N-[2,6-Bis-(1-methylethyl)-phenyl]-N'-(3-phenylpropyl)-harnstoff;
N-[2,6-Bis-(1-methylethyl)-phenyl]-N'-[2-(1-naphthalenyl)-ethyl]-harnstoff;
[R]-N-[2,6-Bis-(1-methylethyl)-phenyl]-N'-[1-(1-naphthalenyl)-ethyl]-harnstoff und
[S]-N-[2,6-Bis-(1-methylethyl)-phenyl]-N'-[1-(1-naphthalenyl)-ethyl]-harnstoff.

11. Verfahren zur Herstellung einer Arzneimittelzubereitung, gekennzeichnet durch Zusetzen einer nach Ansprüche 7 bis 10 hergestellten Verbindung zu einem pharmazeutisch akzeptablen festen oder flüssigen Träger.

## Revendications

1. Un dérivé représenté par la formule structurale: 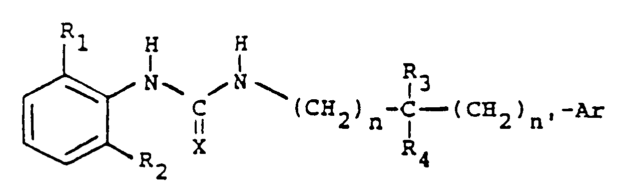 dans laquelle:
R₁ et R₂ sont, indépendamment l'un de l'autre, choisis parmi un radical alkyle comportant entre 1 et 6 atomes de carbone ou un radical alcoxy comportant entre 1 et 6 atomes de carbone;
n est égal à 0 ou représente un nombre entier compris entre 1 et 4;
n' représente un nombre entier compris entre 1 et 4 lorsque n est égal à 0, dans les autres cas, n' est égal à 0;
X représente un atome d'oxygène ou de soufre,
R₃ et R₄ sont, indépendamment l'un de l'autre, choisis parmi un atome d'hydrogène ou un radical alkyle comportant entre 1 et 6 atomes de carbone,
Ar représente un radical phényle, un radical 1- ou 2-naphtyle, un radical 2- 3- ou 4-pyridinyle, 2- ou 3-thiényle, 1- ou 2-naphtyle, phényle non substitué, ou un phényle, 1- ou 2-naphtyle, 2- ou 3-thiényle, 2-, 3- ou 4-pyridinyle substitué par un radical alkyle comportant entre 1 et 6 atomes de carbone,
un radical hydroxy,
un radical alcoxy comportant entre 1 et 6 atomes de carbone,
un radical benzyloxy,
un atome de fluor, de chlore, de brome,
un groupe nitro,
un radical trifluorométhyle,
un groupe -NR₅R₆ dans lequel:
R₅ et R₆ représentent, indépendamment l'un de l'autre, un atome d'hydrogène ou un radical alkyle comportant entre 1 et 6 atomes de carbone, ou
un radical -NH-acétyle;
ou un sel pharmaceutiquement acceptable de ces dérivés.

2. Un dérivé selon la revendication 1, caractérisé en ce que R₁ et R₂ représentent un radical alkyle comportant entre un et six atomes de carbone.

3. Un dérivé selon la revendication 2, caractérisé en ce que R₁ et R₂ représentent un radical 1-méthyléthyle.

4. Un dérivé selon la revendication 1, choisi dans le groupe consistant en:
N-[2,6-bis(1-méthyléthyl)phényl]-N'-(2-phényl-2-propylpentyl)urée;
N-[2,6-bis(1-méthyléthyl)phényl]-N'-(2-éthyl-2-phénylbutyl)urée;
N-[2,6-bis(1-méthyléthyl)phényl]-N'-(2-méthyl-2-phénylpropyl)urée;
N-[2,6-bis(1-méthyléthyl)phényl]-N'-(3,3-diméthyl-2-phénylbutyl)urée;
N-[2,6-bis(1-méthyléthyl)phényl]-N'-(2-butyl-2-phénylhexyl)urée;
N-[2,6-bis(1-méthyléthyl)phényl]-N'-(2-phényléthyl)urée;
N-[2,6-bis(1-méthyléthyl)phényl]-N'-(2-phénylpropyl)urée;
N-[2,6-bis(1-méthyléthyl)phényl]-N'-(3-phénylpropyl)urée;
N-[2,6-bis(1-méthyléthyl)phényl]-N'-(2-(1-naphtalényl)éthyl]urée:
[R]-N-[2,6-bis(1-méthyléthyl)phényl]-N'-[1-(1-naphtalényl)éthyl]urée; et
[S]-N-[2,6-bis(1-méthyléthyl)phényl]-N'-[1-(1-naphtalényl)éthyl]urée.

5. Une composition pharmaceutique comprenant une quantité efficace pour inhiber l'ACAT d'un dérivé selon les revendications 1 à 4, en combinaison avec un support pharmaceutiquement acceptable.

6. L'utilisation d'un dérivé selon les revendications 1 à 4, pour la fabrication de composés pharmaceutiques destinés au traitement de l'hypercholestérolémie ou de l'athérosclérose.

7. Un procédé de préparation d'un dérivé représenté par la formule: 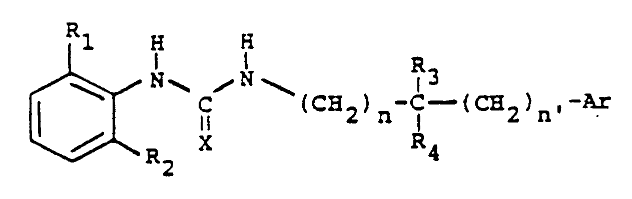 dans laquelle:
R₁ et R₂ sont, indépendamment l'un de l'autre, choisis parmi un radical alkyle comportant entre 1 et 6 atomes de carbone ou un radical alcoxy comportant entre 1 et 6 atomes de carbone;
n est égal à 0 ou représente un nombre entier compris entre 1 et 4;
n' représente un nombre entier compris entre 1 et 4 lorsque n est égal à 0, dans les autres cas, n' est égal à 0;
X représente un atome d'oxygène ou de soufre,
R₃ et R₄ sont, indépendamment l'un de l'autre, choisis parmi un atome d'hydrogène ou un radical alkyle comportant entre 1 et 6 atomes de carbone,
Ar représente un radical phényle, un radical 1- ou 2-naphtyle, un radical 2- 3- ou 4-pyridinyle, 2- ou 3-thiényle, 1- ou 2-naphtyle, phényle non substitué, ou un phényle, 1- ou 2-naphtyle, 2- ou 3-thiényle, 2-, 3- ou 4-pyridinyle substitué par
un radical alkyle comportant entre 1 et 6 atomes de carbone,
un radical hydroxy,
un radical alcoxy comportant entre 1 et 6 atomes de carbone,
un radical benzyloxy,
un atome de fluor, de chlore, de brome,
un groupe nitro,
un radical trifluorométhyle,
un groupe -NR₅R₆ dans lequel:
R₅ et R₆ représentent, indépendamment l'un de l'autre, un atome d'hydrogène ou un radical alkyle comportant entre 1 et 6 atomes de carbone, ou
un radical -NH-acétyle;
ou un sel pharmaceutiquement acceptable de ces dérivés; comprenant les étapes de réaction d'un isocyanate ou d'un thioisocyanate de formule: 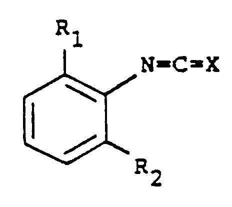 dans laquelle:
R₁, R₂ et X sont tels que définis ci-dessus, avec une amine représentée par la structure: 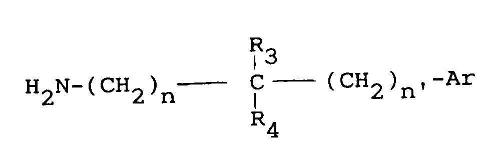
dans laquelle:
n, n', R₃, R₄ et Ar sont tels que définis ci-dessus;
puis la séparation du produit de ladite réaction et la conversion, si cela est nécessaire, dudit produit en un sel pharmaceutiquement acceptable par des procédés classiques.

8. Un procédé de préparation d'un dérivé tel que défini dans la revendication 7, caractérisé en ce que R₁ et R₂ représentent un radical alkyle comportant entre un et six atomes de carbone.

9. Un procédé de préparation d'un dérivé tel que défini dans la revendication 8, caractérisé en ce que R₁ et R₂ correspondent à un radical 1-méthyléthyle.

10. Un procédé de préparation d'un dérivé tel que défini dans la revendication 7, choisi dans le groupe consistant en:
N-[2,6-bis(1-méthyléthyl)phényl]-N'-(2-phényl-2-propylpentyl)urée;
N-[2,6-bis(1-méthyléthyl)phényl]-N'-(2-éthyl-2-phénylbutyl)urée;
N-[2,6-bis(1-méthyléthyl)phényl]-N'-(2-méthyl-2-phénylpropyl)urée;
N-[2,6-bis(1-méthyléthyl)phényl]-N'-(3,3-diméthyl-2-phénylbutyl)urée;
N-[2,6-bis(1-méthyléthyl)phényl]-N'-(2-butyl-2-phénylhexyl)urée;
N-[2,6-bis(1-méthyléthyl)phényl]-N'-(2-phényléthyl)urée;
N-[2,6-bis(1-méthyléthyl)phényl]-N'-(2-phénylpropyl)urée;
N-[2,6-bis(1-méthyléthyl)phényl]-N'-(3-phénylpropyl)urée;
N-[2,6-bis(1-méthyléthyl)phényl]-N'-(2-(1-naphtalényl)éthyl]urée:
[R]-N-[2,6-bis(1-méthyléthyl)phényl]-N'-[1-(1-naphtalényl)éthyl]urée; et
[S]-N-[2,6-bis(1-méthyléthyl)phényl]-N'-[1-(1-naphtalényl)éthyl]urée.

11. Un procédé de préparation d'une composition pharmaceutique caractérisé par l'addition d'un dérivé préparé selon les revendications 7 à 10 à un support liquide ou solide pharmaceutiquement acceptable.
